# EUROPÄISCHE PATENTSCHRIFT

(11) **EP 0 605 763 B1**
(45) Veröffentlichungstag und Bekanntmachung des Hinweises auf die Patenterteilung: **20.08.1997**
(21) Anmeldenummer: 93117757.0
(22) Anmeldetag: 03.11.1993
(51) Int. Cl.: A61B 17/32

(54) **Gewebestanze**
Tissue punch
Poinçonneuse à tissu

(30) Priorität: 05.01.1993 DE 4300064
(43) Veröffentlichungstag der Anmeldung: 13.07.1994
(73) Patentinhaber: Richard Wolf GmbH, 75438 Knittlingen (DE)
(72) Erfinder: Heckele, Helmut, D-75434 Knittlingen (DE)
(74) Vertreter: Wilcken, Thomas, Dipl.-Ing.

(56) Entgegenhaltungen:
- EP-A- 0 150 245
- EP-A- 0 347 170
- EP-A- 0 445 918
- EP-A- 0 530 595
- US-A- 3 902 498
- US-A- 4 340 046
- US-A- 5 106 364

## Beschreibung

Die Erfindung geht aus von einer Gewebestanze mit einem Außenschaft und einem im Außenschaft befindlichen Innenschaft, an dessen distalem Ende eine Öffnung mit einer Schneide vorgesehen ist, die mit einer Gegenschneide am distalen Ende des Außenschaftes so zusammenarbeitet, daß bei Betätigung der Gewebestanze Gewebe, das durch die erwähnte Öffnung in den Innenschaft ragt, durch die gegeneinander bewegten Schneiden abgetrennt wird und durch den Innenschaft absaugbar ist.

Eine Gewebestanze ist beispielsweise aus dem DE-U-78 17 220 bekannt. Dieses Instrument ist mit einem Aufnahmeteil ausgestattet, in welchem die abgetrennten Gewebeteile aufgefangen werden und welches zum Entleeren von dem Instrument abgeschraubt werden kann, was recht umständlich ist. Gleiches gilt sinngemäß für die Handhabung von Kontaktendoskopen (EP-A-0 150 245), die am distalen Ende mit einer Gewebestanze ausgestattet sind, da solche Endoskope jeweils aus dem Körper des Patienten entfernt werden müssen, um das abgetrennte, in einer distalen Aufnahme befindliche Gewebe entnehmen zu können.

Die DE-C-36 30 203 zeigt eine chirurgische Gewebestanze, welche mit einer Absaugeinrichtung zur Entnahme der abgetrennten Gewebeteile ausgestattet ist. Dieses Instrument besteht im wesentlichen aus einem Außenschaft und einem darin axial verschiebbaren Innenschaft. Der Innenschaft ist distalseitig mit einem seitlich offenen Hohlraum ausgeführt, der von einer in Richtung des Außenschaftes gerichteten Schneide begrenzt wird, die an das zu bearbeitende Gewebe so angelegt wird, daß Gewebeteile in diesen Hohlraum hineinragen. Durch Verschieben des Innenschaftes über eine entsprechende Betätigungsvorrichtung gegen den Außenschaft, welcher eine zur Schneide des Innenschaftes entsprechende Gegenschneide aufweist, können dann die in den Hohlraum ragenden Gewebeteile abgeschnitten werden. Mit der Absaugeinrichtung können die Gewebeteile durch den Innenschaft hindurch aus dem Operationsfeld abgesaugt werden. Dabei erfolgt die Absaugung durch Beaufschlagung des Innenschaftes mit einem Unterdruckimpuis, wenn die beiden vorerwähnten Schneiden sich berühren bzw. überdecken. Das abgeschnittene Gewebeteil gelangt auf diese Weise aus dem Instrument in einen Auffangbehälter.

Die vorstehend zitierten Gewebestanzen sind als gerade, starre Instrumente ausgebildet und daher für viele Eingriffe, z. B. im Torax, nur schlecht oder nicht geeignet, da es oft erforderlich ist, daß auch in schwer zugänglichen Bereichen Eingriffe vorzunehmen sind.

Dies ist möglich mit einem biegbaren Instrumentenschaft nach der DE-B-10 19 048 für chirurgische Instrumente, der auch in Verbindung mit Gewebestanzen verwendet werden kann. Dieser Instrumentenschaft besteht im wesentlichen aus einer Federdrahtwendel mit darin gleitend geführtem Führungsschieber und ist an seinem distalen Ende mit einem Anschlußstück für den Anschluß verschiedener Arbeitsmechanismen ausgerüstet. Die Drahtwendel umgibt ein dünnwandiges und biegbares Rohr, welches die Drahtwendel versteift. Auf diese Weise ist der gesamte Instrumentenschaft biegbar und kann vor der Benutzung in die benötigte Form gebracht werden.

Bei Verwendung dieses Instrumentenschaftes als Träger eines Gewebe-Stanzmechanismus ist es jedoch erforderlich, den Instrumentenschaft zur Entnahme der abgetrennten Gewebeteile mit dem Stanzmechanismus aus dem Operationsfeld herauszuziehen.

Es gibt auch Gewebestanzen, bei denen gemäß EP-OS 0 445 918 und US-PS 4 646 738 der Außenschaft starr ist und aus einem proximal geraden Verlauf distalwärts in einen gekrümmten Verlauf übergeht, während der Innenschaft flexibel ist, so daß er sich der Krümmung des Außenschaftes anpassen kann. Der rotierend angetriebene Innenschaft kann axial im Außenschaft verstellt und mit einer an seinem Ende vorgesehenen Schneide mit dem abzutragenden Gewebe in Kontakt gebracht werden, das durch eine Öffnung des Außenschaftes in dessen Innenraum ragt. Das abgetrennte Gewebe wird durch den Innenschaft abgesaugt.

Abgesehen davon, daß Längsbewegungen der schneidenden Teile wegen einer längeren "Offenphase" im allgemeinen effektiver sind als Rotationsbewegungen, haben solche Gewebestanzen einige weitere Nachteile. So bedingen die Maßnahmen, um den Innenschaft beispielsweise durch Materialausschnitte biegsam zu machen, eine unregelmäßige Oberfläche des Schaftkanals mit der Folge, daß abgetragenes Gewebe nicht einwandfrei und nicht reibungsarm durch den Innenschaft abgesaugt werden kann und daß weiterhin Probleme mit der Reinigung und hygienischen Aufbereitung des Instrumentariums entstehen. Außerdem läßt sich ein etwaiger Bruch des Innenschaftes nicht visuell erkennen, und es würde außerdem Schwierigkeiten bereiten, ein distales Fragment des Innenschaftes aus dem Außenschaft zu entfernen. Schließlich ist es dem Operateur auch nicht möglich, wegen der verdeckten Lage der Schneidwerkzeuge deren momentane Stellung zu beobachten.

Durch die Erfindung soll eine Gewebestanze angegeben werden, die frei von solchen Nachteilen ist. Insbesondere soll die Gewebestanze ein problemloses und vollständiges Absaugen des abgetrennten Gewebes gewährleisten und dem Operateur die Möglichkeit geben, die Position und Funktion der Schneiden sowie das jeweils erreichte Behandlungsergebnis beispielsweise endoskopisch überprüfen zu können.

Zur Lösung dieser Aufgabe wird die einleitend erwähnte Gewebestanze erfindungsgemäß so gestaltet, daß der Innenschaft starr ausgebildet ist, im proximalen Bereich gerade verläuft und distalwärts in einen gekrümmten Verlauf übergeht und daß der Außenschaft zumindest im Bereich dieser Krümmung verformbar ist.

Der als Rohr ausgebildete Innenschaft hat einen Kanal mit glatter, reibungsarmer Fläche, durch den Gewebeteile ungehindert abgesaugt werden können und der sich leicht reinigen läßt. Etwaige Schäden an dem durch häufige Biegewechsel relativ stark beanspruchten Außenschaft lassen sich einfach durch Augenschein feststellen. Da die Schneiden von außen frei sichtbar sind, hat der Operateur ferner die Möglichkeit einer ständigen optischen Beobachtung des Behandlungsortes sowie der Position und Funktion der Schneiden, und zwar etwa über ein gesondert zur Anwendung kommendes Endoskop.

Die beispielsweise seitlich gerichtete Öffnung des Innenschaftes kann eine proximalwärts gerichtete Schneide aufweisen, während eine ringförmige Gegenschneide durch das distale Ende des Außenschaftes gebildet wird.

Die mit der am Innenschaft befindliche Schneide zusammenarbeitende Gegenschneide kann auch an einem elastisch verformbaren Schneidenteil ausgebildet sein, der am distalen Ende des Außenschaftes vorgesehen ist und auf einer gekrümmten Bahn verstellt werden kann, die durch Führungen und die distale Form des Innenschaftes bestimmt wird. Eine solche Ausführungsform bietet die Möglichkeit, daß die betreffende Öffnung am Innenschaft nicht nur seitlich, sondern auch distal gerichtet angeordnet werden kann. Im übrigen können die Schneiden an beiden Schäften durch Ränder von Löchern gebildet werden, die in Überdeckung gebracht werden können.

Um eine leichte Verformbarkeit des Außenschaftes zu ermöglichen, ist dieser mit Ausnehmungen versehen, die sich auf den Radien der Krümmung des Innenschaftes gegenüberliegen.

Der Innenschaft weist ein freies Lumen auf, das über seine Öffnung mit der Umgebung in Verbindung steht. Proximalseitig ist der Innenschaft mit einem Anschlußstutzen so verbunden, daß die das freie Lumen bildende Innenbohrung des Innenschaftes mit der Innenbohrung des Anschlußstutzens fluchtet, so daß eine am Anschlußstutzen anzuschließende Absaugeinrichtung abgetragenes Gewebe problemlos und störungsfrei absaugen kann.

Ausführungsbeispiele der erfindungsgemäßen Gewebestanze werden nachstehend anhand der Zeichnung beschrieben. Es zeigen:
- Figur 1: die Seitenansicht einer Gewebestanze, teilweise im Schnitt dargestellt,
- Figur 2: eine Ausführungsform des distalen Endes des Außenschaftes,
- Figur 3: eine weitere Ausführungsform des distalen Endes des Außenschaftes,
- Figur 4: eine Seitenansicht einer motorisch angetriebenen Gewebestanze,
- Figur 5: das distale Ende einer Gewebestanze in Seitenansicht mit einer speziellen Art und Anordnung der Schneiden,
- Figur 6: eine Draufsicht auf das distale Ende der in Figur 5 gezeigten Ausführungsform und
- Figur 7: einen Schnitt entlang der Schnittlinie VII-VII in Figur 5.

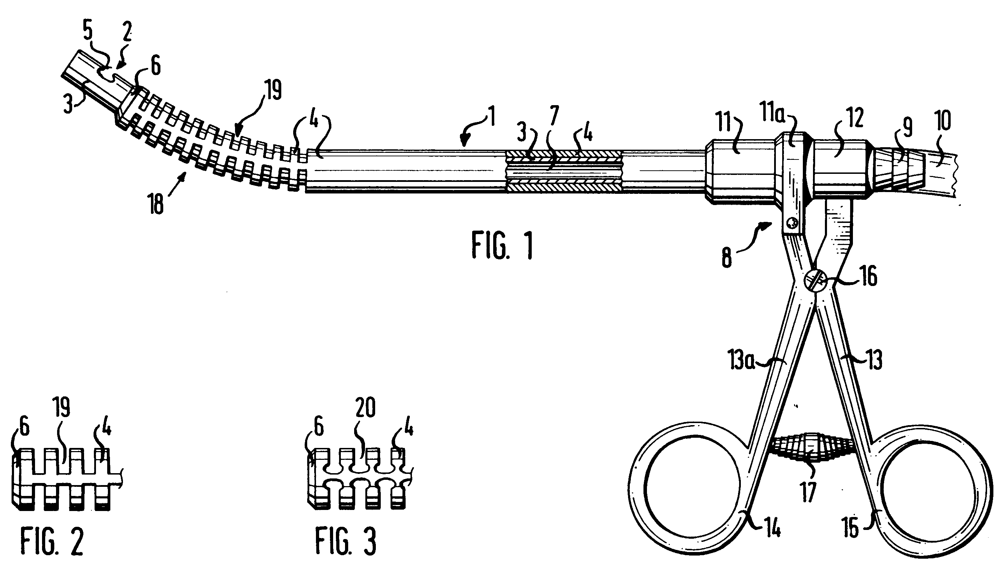

Die Gewebestanze 1 gemäß Figur 1 besteht aus einem am distalen Ende mit einer seitlichen Öffnung 2 versehenen Innenschaft 3, auf dem ein Außenschaft 4 axial bewegbar ist. Die Öffnung 2 weist eine Schneide 5 auf, die beim Stanz- oder Schneidvorgang mit einer Schneide 6 am distalen Ende des Außenschaftes 4 zusammenwirkt. Die Schneide 6 wird durch das scharfe Ende des Außenschaftes 4 gebildet. Der Innenschaft 3 weist ein freies Lumen 7 auf, mit dem die Öffnung 2 in Verbindung steht, und ist proximalseitig mit einem Anschlußstutzen 9 versehen, an dem ein Absaugschlauch 10 angeschlossen werden kann. Die Anordnung des Anschlußstutzens 9 ist dabei so getroffen, daß dessen Innenbohrung mit dem freien Lumen des Innenschaftes 3 fluchtet, so daß dadurch ein Absaugkanal gebildet wird, der sich von der seitlichen Öffnung 2 bis zum proximalen Instrumentenende erstreckt. Der Absaugschlauch 10 führt zu einer nicht dargestellten Absaugvorrichtung.

Die Handhabe 8 weist eine Hülse 11 auf, die einerseits mit dem Außenschaft 4 und andererseits über ein Verstärkungsteil 11a mit einem Scherenschenkel 13a der Handhabe verbunden ist. Proximalseitig der Hülse 11 sind der Innenschaft 3 und der Anschlußstutzen 9 in einer zweiten Hülse 12 festgelegt, welche ihrerseits mit einem zweiten Scherenschenkel 13 in Verbindung steht. Die beiden Scherenschenkel 13 und 13a enden jeweils in Scherengriffen 14 und 15 und sind über eine Schraube 16 gelenkig miteinander verbunden. Eine als zweiseitige Kegelfeder ausgebildete Druckfeder 17 hält die Scherenschenkel 13 und 13a auseinander, so daß die Öffnung 2 des Innenschaftes 3 vollständig distalseitig aus dem Außenschaft 4 der Gewebestanze 1 herausragt. In dieser Stellung liegt die Hülse 11 mit ihrem Verstärkungsteil 11a am distalen Ende der Hülse 12 an, die einen größeren Außendurchmesser als die Hülse 11 aufweist und somit als Anschlag für das Verstärkungsteil 11a der Hülse 11 dient.

Die Gewebestanze 1 weist in ihrem distalen Abschnitt einen gekrümmten Bereich 18 auf. Die Krümmung ist bedingt durch den entsprechend gekrümmten Innenschaft 3, auf dem sich der Außenschaft 4 abstützt. Dieser kann sich der Krümmung des Innenschaftes 3 dadurch anpassen, daß er mit sich gegenüberliegenden Ausnehmungen 19 in Form von radialen Einschnitten versehen ist, die lediglich eine schmale Materialbrücke zwischen sich belassen. Die Flexibilität dieses biegsamen Bereiches 18 des Außenschaftes 4 kann durch die Anzahl, Anordnung und Geometrie der Ausnehmungen 19 bestimmt werden.

So zeigt die Figur 2 eine erste Ausführungsform dieses Biegebereiches, bei der die Ausnehmungen 19 durch einfache Säge- oder Fräseinschnitte mit eckigem Profil hergestellt sind.

Eine weitere Ausführungsform zeigt die Figur 3, bei der die Ausnehmungen 20 an ihren Enden eine Form aufweisen, die zwar aufwendig in der Herstellung ist, aber besonders enge Biegungen verkraftet. Die Anzahl der mit dieser Ausführung erreichbaren Biegewechsel ist gegenüber der Ausführungsform nach Figur 2 höher, da diese Ausführung kerbwirkungsfrei ist.

Zum Abschneiden von Gewebe oder Gewebeteilen wird die Gewebestanze so gegen das zu entfernende Gewebe angelegt, daß Teile davon in die Öffnung 2 des Innenschaftes 3 hineinragen. Durch Betätigen der Scherengriffe 13 und 13a gegen die Druckfeder 17 wird der Außenschaft 4 mit seiner Schneide 6 gegen die Öffnung 2 geschoben, bis die Schneide 5 der Öffnung 2 und die Schneide 6 des Außenschaftes 4 scheren, so daß das zu entfernende Gewebeteil abgetrennt wird. Dieses gelangt dadurch in das freie Lumen 7 in dem Innenschaft 3 und kann über den Absaugschlauch 10 aus dem Innenschaft 3 in die Absaugeinrichtung abgesaugt werden.

Der Innenschaft 3 wie auch der Außenschaft 4 sind vorzugsweise aus Metall gefertigt, so daß die Schneiden 5 bzw. 6 direkt an diesen Teilen ausbildbar sind. Es ist jedoch auch möglich, den Außenschaft 4 aus Kunststoff zu fertigen, wobei dann die Schneide 6 durch einen gesonderten Einsatz aus Metall gebildet werden kann.

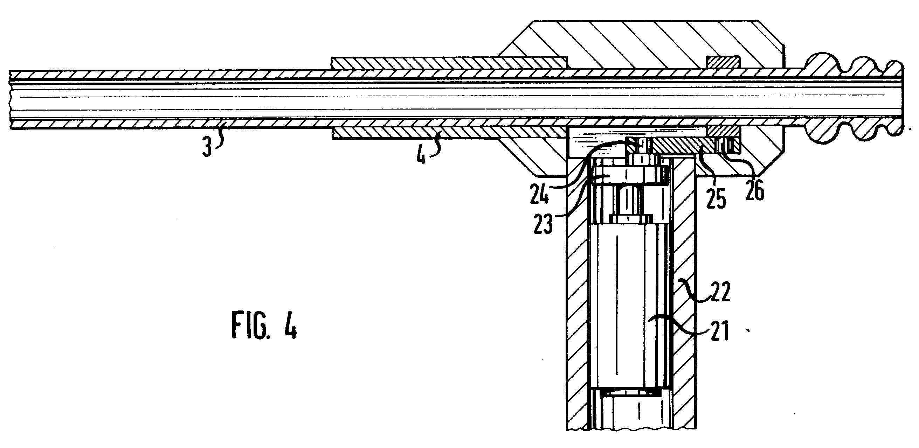

Die Figur 4 zeigt in vereinfachter Darstellung eine motorisch angetriebene Gewebestanze mit einem Elektromotor 21 in einem Gehäuse 22, welches einen Handgriff zum Halten der Gewebestanze bildet. Auf einer vom Motor drehbeweglich angetriebenen Scheibe 23 sitzt exzentrisch zur Drehachse ein Zapfen 24, der in eine Bohrung eines Lenkers 25 greift. In eine weitere Bohrung dieses Lenkers faßt ein Mitnehmerzapfen 26, der am Innenschaft 3 befestigt ist.

Durch den so gebildeten Exzenterantrieb wird bei laufendem Motor 21 der Innenschaft 3 relativ zum feststehenden bzw. festgehaltenen Außenschaft 4 in oszillierende Bewegungen versetzt, wobei in entsprechender Weise wie bei der vorher im Zusammenhang mit Figur 1 beschriebenen Handbetätigung die Schneiden der Gewebestanze zum Abtrennen von Gewebe verstellt werden.

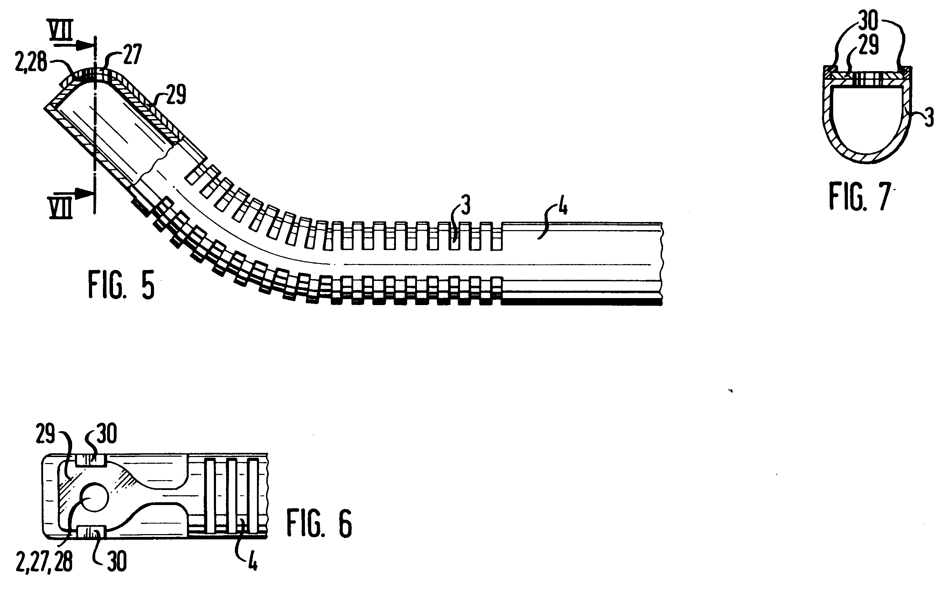

Bei dem in den Figuren 5 bis 7 gezeigten Ausführungsbeispiel sind Bauteile, die den in den anderen Figuren dargestellten entsprechen, mit gleichen Bezugszeichen versehen. Auch diese Gewebestanze hat dementsprechend einen starren, am distalen Endbereich gekrümmten Innenschaft 3 und einen Außenschaft 4, der wenigstens in dem Bereich verformbar ist, wo er bei Axialbewegungen der Krümmung des Innenschaftes folgen muß.

Die vorher als Gegenschneide bezeichnete Schneide des Außenschaftes 4 und die Schneide des Innenschaftes 3 sind in diesem Fall als Ränder von Löchern 27, 28 ausgebildet, die zur Freigabe der Öffnung 2 gemäß Figur 5 in Überdeckung gebracht werden können, und zwar durch entsprechende Relativbewegung beider Schäfte.

Das Loch 27 des Außenschaftes 4 befindet sich in einem elastisch biegsamen Schneidenteil 29, das ein distaler Fortsatz des Außenschaftes ist und der Darstellung in Figur 5 entsprechend auf einer gekrümmten Bahn bewegbar ist, und zwar bei axialer Verstellung des Außenschaftes relativ zum Innenschaft, um die schneidenden Ränder der Löcher 27, 28 scheren zu lassen und das durch die Öffnung 2 in den Innenschaft ragende Gewebe abzutrennen.

Die Bewegungsbahn des Schneidenteils 29 ist durch die benachbarte Kontur des Innenschaftes 3 und durch Führungen 30 an dessen distalem Ende vorgegeben, wobei die Führungen seitlich über das Schneidenteil 29 greifen und dieses in Anlage mit dem Innenschaft halten.

Zweckmäßigerweise ist das Schneidenteil so wie aus Figur 5 ersichtlich vorgeformt, so daß bei der Betätigung der Gewebestanze vergleichsweise geringe elastische Verformungen bzw. mechanische Beanspruchungen des Schneidenteils auftreten werden. Im übrigen bietet diese Lösung den Vorteil, daß die Öffnung 2 nicht seitlich gerichtet wie etwa in Figur 1 gezeigt sein muß, sondern auch distal oder wenigstens mit distaler Komponente gerichtet vorgesehen werden kann.

## Patentansprüche

1. Gewebestanze mit einem Außenschaft (4) und einem im Außenschaft befindlichen Innenschaft (3), an dessen distalem Ende eine Öffnung (2) mit einer Schneide (5) vorgesehen ist, die mit einer Gegenschneide (6) am distalen Ende des Außenschaftes (4) so zusammenarbeitet, daß bei Betätigung der Gewebestanze Gewebe, das durch die erwähnte Öffnung (2) in den Innenschaft (3) ragt, durch die gegeneinander bewegten Schneiden (5, 6) abgetrennt wird und durch den Innenschaft absaugbar ist, dadurch gekennzeichnet, daß der Innenschaft starr ausgebildet ist, im proximalen Bereich gerade verläuft und distalwärts in einen gekrümmten Verlauf übergeht und daß der Außenschaft (4) zumindest im Bereich dieser Krümmung verformbar ist.

2. Gewebestanze nach Anspruch 1, dadurch gekennzeichnet, daß die Öffnung (2) des Innenschaftes (3) seitlich gerichtet ist und die Schneide (5) aufweist, welche proximalwärts gerichtet ist, und daß die Gegenschneide (6) das distale Ende des Außenschaftes (4) bildet.

3. Gewebestanze nach Anspruch 1, dadurch gekennzeichnet, daß die Gegenschneide an einem elastisch verformbaren Schneidenteil (29) ausgebildet ist, welches als Fortsatz am distalen Ende des Außenschaftes (4) vorgesehen und auf einer gekrümmten Bahn verstellbar ist, die durch Führungen (30) am distalen Ende des Innenschaftes (3) bestimmt ist.

4. Gewebestanze nach Anspruch 3, dadurch gekennzeichnet, daß die Schneiden an beiden Schäften durch Ränder von Löchern (27, 28) gebildet sind und daß die Löcher in Überdeckung bringbar sind.

5. Gewebestanze nach einem der Ansprüche 1 bis 4, dadurch gekennzeichnet, daß der Außenschaft (4) mit Ausnehmungen (19 bzw. 20) versehen ist, die sich auf den Radien der Innenschaftkrümmung gegenüberliegen.

6. Gewebestanze nach einem der Ansprüche 1 bis 5, dadurch gekennzeichnet, daß der Innenschaft (3) ein freies Lumen (7) aufweist, mit welchem seine Öffnung (2) verbunden ist, und daß der Innenschaft (3) proximalseitig mit einem Anschlußstutzen (9) derart verbunden ist, daß die das freie Lumen (7) bildende Innenbohrung des Innenschaftes (3) mit der Innenbohrung des Anschlußstutzens (9) fluchtet.

7. Gewebestanze nach einem der Ansprüche 1 bis 6, dadurch gekennzeichnet, daß der Außenschaft (4) und der Innenschaft (3) im jeweils proximalen Endbereich, d. h. bei letzterem im Bereich des Anschlußstutzens (9), je mit einem Scherenschenkel (13, 13a) verbunden sind, die ein Handhabenteil (8) zur gegenseitigen axialen Verschiebung von Außenschaft (4) und Innenschaft (3) bilden.

8. Gewebestanze nach Anspruch 7, dadurch gekennzeichnet, daß eine an den Scherenschenkeln (13 bzw. 13a) angreifende Druckfeder (17) vorgesehen ist, die den Innenschaft (3) im Verhältnis zum Außenschaft (4) so in einer Lage hält, daß die seitliche Öffnung (2) des Innenschaftes (3) distalseitig vollständig aus dem Außenschaft (4) herausragt.

9. Gewebestanze nach einem der Ansprüche 1 bis 8, dadurch gekennzeichnet, daß einer der Schäfte (3; 4) mit einem motorischen Antrieb (21) relativ zum anderen Schaft (4; 3) in oszillierende Bewegungen versetzbar ist.

10. Gewebestanze nach Anspruch 9, dadurch gekennzeichnet, daß der Antrieb (21) in einem einen Handgriff bildenden Gehäuse (22) angeordnet ist.

## Claims

1. A tissue punch having an outer shank (4) and located in this outer shank, an inner shank (3) at the distal end of which there is provided an opening (2) with a cutter (5) which so cooperates with a counter cutter (6) that on operation of the tissue punch, tissue, which protrudes through said opening (2) into the inner shank (3), is severed by the cutters (5, 6) moving against each other and can be aspirated through the inner shank, characterised in that the inner shank is rigidly formed, runs straight in the proximal region, and distally blends into an arcuate course and that the outer shank (4) is deformable at least in the region of this curvature.

2. A tissue punch according to claim 1, characterised in that the opening (2) of the inner shank (3) is directed laterally and comprises the cutter (5) which is directed proximally, and that the counter cutter (6) forms the distal end of the outer shank (4).

3. A tissue punch according to claim 1, characterised in that the counter cutter is formed on an elastically deformable cutting part (29) which is provided as an extension on the distal end of the outer shank (4) and is adjustable on a curved path which is determined by the guides (30) at the distal end of the inner shank (3).

4. A tissue punch according to claim 3, characterised in that the cutters on both shanks are formed by the edges of holes (27, 28) and that the holes may be brought to overlap.

5. A tissue punch according to one of claims 1 to 4, characterised in that the outer shank (4) is provided with recesses (19 or 20) which lie opposite the radii of the curvature of the inner shank 6.

6. A tissue punch according to one of claims 1 to 5, characterised in that the inner shank (3) comprises a free lumen (7) to which the opening (2) of said inner shank is connected, and that the inner shank, on the proximal side, is connected to a connecting piece (9) in such a manner that the inner bore, forming the free lumen (7), of the inner shank (3) is flush with the inner bore of the connecting piece (9).

7. A tissue punch according to one of claims 1 to 6, characterised in that the outer shank (4) and the inner shank (3)
in each case at the proximal end region, i.e. with the latter in the region of the connecting piece (9), each are connnected to a scissor arm (13, 13a) which form a handle part (8) for the mutual axial displacement of the outer shank (4) and the inner shank (3).

8. A tissue punch according to claim 7, characterised in that
there is provided a compression spring (17) engaging on the scissor arms (13 or 13a), said spring holding the inner shank (3) in such a position in relation to the outer shank (4) that the lateral opening (2) of the inner shank (3) completely protrudes from the outer shank (4) on the distal side.

9. A tissue punch according to one of claims 1 to 8, characterised in that one of the shanks (3; 4) may be displaced with oscillating movement relative to the other shank (4; 3) by way of a motor drive (21).

10. A tissue punch according to claim 1, characterised in that the drive (21) is arranged in a housing (22) forming a handle.

## Revendications

1. Poinçonneuse pour tissu comportant un tube extérieur (4) et un tube intérieur (3) situé dans le tube extérieur et sur l'extrémité distale duquel est prévue une ouverture (2) comportant un tranchant (5), qui coopère avec un tranchant antagoniste (6) situé sur l'extrémité distale du tube extérieur (4), d'une façon telle que, lors de l'actionnement de la poinçonneuse pour tissu, du tissu, qui pénètre dans le tube intérieur (3) à travers l'ouverture mentionnée (2), est sectionné par les tranchants (5,6) déplacés l'un par rapport à l'autre et peut être aspiré à travers le tube intérieur, caractérisée en ce que le tube intérieur est d'une conception rigide, possède une forme rectiligne au niveau de la partie proximale et se prolonge, vers le côté distal, sous une forme incurvée et en ce que le tube extérieur (4) peut être déformé au moins dans la région de cette partie incurvée.

2. Poinçonneuse pour tissu selon la revendication 1, caractérisée en ce que l'ouverture (2) du tube intérieur (3) est orientée latéralement et comporte le tranchant (5) qui est orienté vers le côté proximal, et en ce que le tranchant antagoniste (6) forme l'extrémité distale du tube extérieur (4).

3. Poinçonneuse pour tissu selon la revendication 1, caractérisée en ce que le tranchant antagoniste est conçu sous la forme d'un tranchant (29) déformable élastiquement, qui est prévu sous forme d'un prolongement à l'extrémité distale du tube extérieur (4) et est déplaçable sur une trajectoire incurvée, qui est déterminée par des guides (30) situés à l'extrémité distale du tube intérieur (3).

4. Poinçonneuse pour tissu selon la revendication 3, caractérisée en ce que les tranchants sont, sur les deux tubes, formés par des bords de trous (27,28) et en ce que les trous peuvent être amenés en superposition.

5. Poinçonneuse pour tissu selon l'une des revendications 1 à 4, caractérisée en ce que le tube extérieur (4) comporte des évidements (19, 20), qui sont situés en vis-à-vis sur les zones arrondies de la partie incurvée du tube intérieur.

6. Poinçonneuse pour tissu selon l'une des revendications 1 à 5, caractérisée en ce que le tube intérieur (3) présente une lumière dégagée (7), à laquelle son ouverture (2) est reliée, et en ce que le tube intérieur (3) est relié, côté proximal, à un embout de raccordement (9) de telle sorte que le perçage intérieur, qui constitue la lumière dégagée (7), du tube intérieur (3) soit aligné avec le perçage intérieur de l'embout de raccordement (9).

7. Poinçonneuse pour tissu selon l'une des revendications 1 à 6, caractérisée en ce que le tube extérieur (4) et le tube intérieur (3) sont reliés, respectivement dans la partie d'extrémité proximale, c'est-à-dire au niveau de cette dernière dans la région de l'embout de raccordement (9), à des branches respectives de ciseaux (13,13a), qui forment un élément de manipulation (8) servant à déplacer axialement le tube extérieur (4) et le tube intérieur (3) l'un par rapport à l'autre.

8. Poinçonneuse pour tissu selon la revendication 7, caractérisée en ce qu'il est prévu un ressort de poussée (17), qui s'appuie sur les branches de ciseaux (13,13a) et qui maintient le tube intérieur (3) dans une position telle, par rapport au tube extérieur (4), que l'ouverture latérale (2) du tube intérieur (3) sorte complètement, côté distal, du tube extérieur (4).

9. Poinçonneuse pour tissu selon l'une des revendications 1 à 8, caractérisée en ce que l'un des tubes (3;4) peut être animé de mouvements oscillatoires par rapport à l'autre tube (4;3) à l'aide d'un dispositif d'entraînement motorisé (21).

10. Poinçonneuse pour tissu selon la revendication 9, caractérisée en ce que le dispositif d'entraînement (21) est disposé dans un boîtier (22) constituant une poignée.
